# EUROPEAN PATENT APPLICATION

(11) **EP 2 111 889 A1**
(43) Date of publication of application: **28.10.2009**
(21) Application number: 08381015.0
(22) Date of filing: 25.04.2008
(51) Int. Cl.: A61N 1/30

(54) **Roll-on device for electrotherapy machine**

(71) Applicant: Font I Mas, Juan Carlos, 08850 Gava (ES)
(72) Inventor: Font I Mas, Juan Carlos, 08850 Gava (ES)
(74) Representative: Martin Santos, Victoria Sofia

(57) **Abstract**

This invention refers to a roll-on device specially indicated for its use with an electrotherapy machine. The roll-on device which is the object of this invention is of the type comprising a container for holding the product, a main body, a roller element for dispensing the product housed at an end of the main body, a dispensing chamber included in the main body which connects the container to the roller element and means of connection to an energy supply component. The roll-on device in this invention is characterised by the fact that the means of connection to the energy supply component comprise a housing for the aforementioned component situated outside the main body, with the main body made from conductive material so that energy, normally electricity, is transmitted through the body, to the roller element.

## Description

### OBJECT OF THE INVENTION

This invention refers to a roll-on device specially indicated for use with an electrotherapy machine.

The roll-on device which is the object of this invention is of the type comprising a container for holding the product, a main body, a roller element for dispensing the product housed at one end of the main body, a dispensing chamber included in the main body which connects the container to the roller element, and means of connection to an energy supply component.

The roll-on device in this invention is characterised by the fact that the means of connection to the energy supply component comprise a housing for the aforementioned component situated outside the main body, with the main body made from conductive material so that energy, normally electricity, is transmitted through the body, to the roller element.

### BACKGROUND TO THE INVENTION

Electrotherapy machines are known which produce the effect of electrophoresis, or iontophoresis and electroporation. Normally they are provided with at least one pair of electrodes with one pole applied in the zone of the body to be treated, and another pole closing the circuit of the electric continuous current through the patient's body. These machines are able to increase penetration of the cosmetic which is presented in the form of ionic molecules

It is known that the electrode in contact with the patient's body is a roll-on type device which in addition may also be provided with a product dispensing function, and it may be provided with means which permit coupling of a container which holds the solution to be applied.

Patent EP1178854 is cited as background disclosing a container for a gel suspension of molecules or substances to be administered, requiring application of energy which stimulates the penetration of the molecules to be transported through the epidermis and beyond the dermal barrier of the skin. The device comprises a combination of a container for the product to be administered, a dispensing element through which the product will be administered, and an energy supply component situated adjacent to said dispensing element.

However, the aforementioned device requires that the energy supply component, that is, the end of the electrode is included in the interior of the dispensing chamber and in direct contact with the roller dispensing element. This fact has the disadvantage that the end of the electrode may suffer deterioration as it is in permanent contact with both the fluid to be applied and the rolling element, with its replacement being problematic as it is necessary to dismantle the roll-on in order to access and replace said electrode.

This invention resolves the previous problem by means of a device which prevents deterioration of the electrode, in addition to permitting greater facility of use and handling thereof.

### DESCRIPTION OF THE INVENTION

This invention refers to a roll-on device especially indicated for its use as a dispensing element in an electrotherapy machine.

The electrotherapy machine is used to increase absorption through the skin of cosmetic products, so that cosmetic products applied are able to reach deep levels of the skin, increasing the effectiveness thereof. The machine normally comprises a first pole of a pair of electrodes situated in a head with the capacity to move over the skin of the patient and a second pole of the pair of electrodes which closes the electrical circuit on the body of the patient.

In this case the head is a roll-on device of the type which includes:
- a container for holding the product. The container may be easily interchangeable, so that once the content has finished it can be replaced with a new one, or either is possible to change containers holding different lotions indicated for different types of treatment;
- a main body, which may include means for permitting easy coupling of the product container;
- a roller element for dispensing the product, housed in one end of the main body;
- a dispensing body, including a main body which connects the container to the roller element, which may be cylindrical or spherical; and
- means of connection to an energy supply component. The energy supply component shall normally be the end of an electrode connected by its other end to a source of electrical energy.

This invention is **characterised in that** the means for connection to the energy supply component include housing for the aforementioned component in the exterior of the main body. The main body is made from conductive material, so that energy, normally electrical, is transmitted through the body to the roller element which is the contact with the patient's body, and therefore it has the function of transmitting that electrical energy to the epidermis, thus increasing the penetration of the product.

### DESCRIPTION OF THE DRAWINGS

The present descriptive report is complemented by a set of plans illustrating a preferred embodiment of the invention, but which is in no way restrictive.

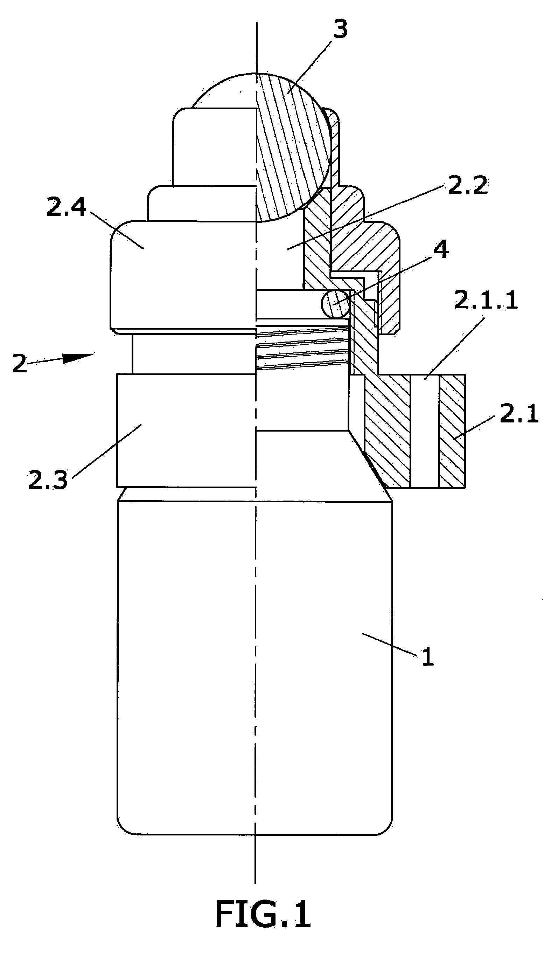

Figure 1 is an example of an embodiment of the roll-on device which is the object of the invention.

### PREFERRED EMBODIMENT OF THE INVENTION

Figure 1 shows a frontal view, together with a section of an example of an embodiment of the roll-on device which is the object of the invention.

The head comprises a container (1) which contains the product to be applied, a main body (2) at whose opposite end to the container coupling (1)a roller element (3) is situated for dispensing the product. The main body (2) comprises a dispensing chamber (2.2) which connects the container (1) to the roller element (3).

The container (1) and the main body (2) are joined by a threaded part.

The means of connection to the energy supply component include a housing (2.1.1) for insertion of the component, for example of the end of an electrode, that is, a cable. This housing is situated on the external surface of the main body (2) in a projection (2.1) of the body (2).

Both the main body (2) and the roller element (3) are made from conductive material, for example, steel.

In the preferred embodiment the main body (2) comprises two semi-bodies (2.3, 2.4) screwed together. The first semi-body (2.3) is in contact with the container (1) and extends to the proximity of the roller element (3) defining the lateral walls of the dispensing chamber (2.2). The second semi-body (2.4) retains the roller element (3).

Between the end of the container (1) in contact with the main body (2) and the first semi-body (2.3) there is a sealing joint (4).

## Claims

1. Roll-on device for an electrotherapy device comprising a container (1) for holding the product to be applied n connection with a main body (2) where, at the end opposite the container (1) coupling, a roller element(3) is situated for dispensing the product, a dispensation chamber (2.2) included in the main body (2) which connects the container (1) to the roller element (3) and means of connection to an electrical energy supply component **characterised in that** the means of connection to the energy supply component comprise a housing (2.1.1) for insertion of the aforementioned component, with this housing situated on the external surface of the main body(2), with the main body (2) being made from conductive material.

2. Roll-on device for an electrotherapy machine according to claim 1, **characterised in that** the housing (2.1.1) is situated on a projection (2.1) of the main body (2).

3. Roll-on device for an electrotherapy machine according to claim 1, **characterised in that** the main body (2) comprises two semi-bodies (2.3, 2.4) screwed together.

4. Roll-on device for an electricity machine, according to claim 3, **characterised in that** the first semi-body (2.3) is in contact with the container (1) and extends to the proximity of the roller element (3) defining the lateral walls of the dispensing chamber (2.2).

5. Roll-on device for an electrotherapy machine according to claim 3, **characterised in that** the second semi-body (2.4) retains the roller element (3).

6. Roll-on device for electrotherapy machine according to claim 3, **characterised in that** between the end of the container (1) in contact with the main body (2) and the first semi-body (2.3) there is a sealing joint (4).

7. Roll-on device for an electrotherapy machine according to claim 1, **characterised in that** the container (1) and the main body (2)are screwed together.

8. Roll-on device for an electrotherapy machine according to claim 1, **characterised in that** both the main body (2) and the roller element (3) are made from steel.
